**Europäisches Patentamt**

(19) **European Patent Office**

**Office européen des brevets**

(11) Veröffentlichungsnummer : **0 011 842**
**B1**

(12) **EUROPÄISCHE PATENTSCHRIFT**

(45) Veröffentlichungstag der Patentschrift :
26.01.83

(51) Int. Cl.³ : **C 07 C 27/02, C 07 C 27/20**

(21) Anmeldenummer : **79104690.7**

(22) Anmeldetag : **26.11.79**

(54) **Verfahren zum Abbau von Ameisensäureestern.**

(30) Priorität : **29.11.78 DE 2851515**

(43) Veröffentlichungstag der Anmeldung :
**11.06.80 Patentblatt 80/12**

(45) Bekanntmachung des Hinweises auf die Patenterteilung : **26.01.83 Patentblatt 83/04**

(84) Benannte Vertragsstaaten :
**FR IT NL**

(56) Entgegenhaltungen :
**US A 2 564 130**
**US A 2 894 990**

(73) Patentinhaber : **Ruhrchemie Aktiengesellschaft**
**Bruchstrasse 219**
**D-4200 Oberhausen 13 (DE)**

(72) Erfinder : **Tomuschat, Hans Joachim H.A., Dr.**
**Dipl.-Chem.**
**Burgstrasse 22**
**D-4200 Oberhausen 13 (DE)**

(74) Vertreter : **Reichelt, Karl-Heinz, Dr.**
**m. Br. Ruhrchemie Aktiengesellschaft Abt. PLD Postfach 13 01 60**
**D-4200 Oberhausen 13 (DE)**

Verfahren zum Abbau von Ameisensäureestern

Die vorliegende Erfindung betrifft ein Verfahren zum Abbau von Ameisensäureestern die als Nebenprodukte bei der Umsetzung von Olefinen mit 4 bis 12 Kohlenstoffatomen mit Kohlenmonoxid und Wasserstoff in Gegenwart Carbonylverbindungen bildender Metalle, wie Kobalt oder Rhodium, entstehen (Hydroformylierung) in Ameisensäure und den zugehörigen Alkohol.

Bei der Umsetzung von Olefinen mit Kohlenmonoxid und Wasserstoff, die in Gegenwart von Metallen der 8. Gruppe des Periodensystems abläuft, werden neben Aldehyden und Alkoholen stets auch Ameisensäureester gebildet. Als Alkoholkomponente enthalten diese Ameisensäureester überwiegend den aus dem Einsatzolefin entstandenen Alkohol, der ein Kohlenstoffatom mehr als dieses Olefin aufweist.

Die Ameisensäureester sind unerwünschte Nebenprodukte der Hydroformylierungsreaktion, da sie einen Teil der Alkohole binden und außerdem die Reingewinnung der Alkohole erschweren. So ist eine destillative Trennung der Alkohole und ihrer Ameisensäureester wegen der geringen Siedepunktdifferenz und der Bildung von azeotropen Gemischen miteinander praktisch kaum möglich. Da die Destillation jedoch die einzig brauchbare technische Aufarbeitungsmethode für die Reaktionsprodukte ist, müssen diese Ester vor der Reindestillation der Alkohole entfernt werden.

Es sind bereits mehrere Verfahren bekannt, deren Ziel die Entfernung von Ameisensäureestern aus ihren Gemischen mit Alkoholen ist.

Nach dem in der DE-AS 11 06 198 beschriebenen Verfahren sollen durch Oxosynthese hergestellte Alkohole zur Verbesserung ihrer Qualität mit Mineralsäuren oder einem sauer reagierenden Salz einer Mineralsäure behandelt werden. Bei dem in der DE-AS 11 48 221 beschriebenen Verfahren werden statt Mineralsäuren Lewis-Säuren verwendet. Diese Verfahren führen zu einer Verminderung der Alkoholausbeute und sind wegen des Auftretens von Korrosionsproblemen nur beschränkt anwendbar.

Entsprechend der in der DE-AS 10 85 573 beschriebenen Arbeitsweise behandelt man Alkohole aus der Oxosynthese zur Beseitigung von Estern und anderen Verunreinigungen und zur Verbesserung ihrer Qualität mit alkoholischer Lauge. Hierbei muß das Alkali in stöchiometrischer Menge angewandt werden. Darüber hinaus dürfen die Alkohole keine nennenswerten Mengen an Aldehyden enthalten, da sich diese in Gegenwart von Alkali umsetzen und als Wertprodukte verlorengehen.

Nach weiteren bekannten Verfahren kann die Spaltung von Ameisensäureestern durch thermische Behandlung mit einer wässrigen Lösung von Alkalisalzen einer organischen Säure (DE-PS 12 58 855), durch Umsetzung an einem Aluminiumoxid enthaltenden Katalysator (DE-PS 18 17 051) oder durch Hydrierung in Gegenwart von Nickelkatalysatoren erfolgen. Diese Verfahren liefern teilweise gute Ergebnisse, erfordern aber die Bereitstellung zusätzlicher Anlagen sowie Materialien und erlauben daher nicht immer eine wirtschaftliche Lösung des vorhandenen Problems.

Die US-PS 2 564 130 beschreibt ein Verfahren zur Herstellung von Butanolen, wobei die Bildung von Butylformiat dadurch verringert werden soll, daß Wasser als Lösungsmittel verwendet wird. Dies bedeutet, daß Wasser in sehr großen Mengen bei hohen Temperaturen und hohen Drücken eingesetzt werden müssen. Besonders nachteilig wirkt sich das durch den Wassergehalt entsprechend gemindertem Reaktionvolumen aus, das nur einen erheblich geringeren Durchsatz zuläßt.

Es bestand daher die Aufgabe, ein Verfahren zur Spaltung von Ameisensäureestern zu entwickeln, das die aufgezeigten Schwierigkeiten umgeht.

Die Erfindung besteht in einem Verfahren zum Abbau von Ameisensäureestern oder Ameisensäureester enthaltenden Gemischen, die bei der Umsetzung von Olefinen mit 4 bis 12 Kohlenstoffatomen mit Kohlenmonoxid und Wasserstoff bei 100 bis 250 °C und 100 bis 300 bar in Gegenwart von Kobaltkatalysatoren erhalten werden und nach Abtrennung der Katalysatoren und der leichter flüchtigen Reaktionsprodukte, insbesondere der Aldehyde als Nachlauffraktion anfallen. Es ist dadurch gekennzeichnet, daß die Ameisensäureester bzw. die Ameisensäureester enthaltenden Gemische in die Synthesestufe zurückgeführt und zusammen mit Olefin, Kohlenmonoxid und Wasserstoff in Gegenwart von 2 bis 5 Gew.-% Wasser, bezogen auf das Einsatzgemisch, bei Temperaturen von 100 bis 180 °C umgesetzt werden.

Durch Anwendung des erfindungsgemäßen Verfahrens wird die Ausbeute an Alkoholen hoher Reinheit, bezogen auf eingesetztes Olefin, deutlich verbessert.

Der besondere Vorteil des neuen Prozesses ist darin zu sehen, daß zusätzliche Anlagen zu seiner Durchführung nicht erforderlich sind, vielmehr mit den vorhandenen Syntheseeinrichtungen gearbeitet werden kann.

Als Einsatzmaterial finden Ameisensäureester Anwendung, die bei der Hydroformylierung von Olefinen mit 4 bis 12 Kohlenstoffatomen wie Hexen, Octen, Nonen als Nebenprodukte entstehen, z.B. die Ameisensäureester des Hexyl-, Octyl-, Nonyl-Alkohols. Diese Ester können in reiner Form eingesetzt werden. Die erfindungsgemäße Arbeitsweise erlaubt es jedoch auch, Gemische einzusetzen, in denen die Ester enthalten sind. Derartige Gemische fallen bei der Aufarbeitung der Reaktionsprodukte an, die aus den oben genannten Olefinen bei der Umsetzung mit Kohlenmonoxid und Wasserstoff in Gegenwart von Kobaltkatalysatoren gebildet werden. Üblicherweise wird aus dem Reaktionsprodukt zunächst der Katalysator abgetrennt. Hierzu behandelt man es z.B. mit Wasser oder Wasserdampf, deren Wirkung man durch Zusatz von Säure und/oder Oxidationsmittel unterstützen kann. Aus dem verbleibenden Gemisch werden darauf die niedrigsiedenden Aldehyde, die eigentlichen Wertprodukte der Reaktion abgetrennt. Die Nachlauffraktion — sie fällt in einer Menge von ca. 15 Gew.-%, bezogen auf das rohe Reaktionsprodukt an — enthält neben Alkoholen (ca. 60 Gew.-%) die aus den Aldehyden durch gleichzeitig ablaufende Hydrierung gebildet werden, die Ameisensäureester (ca. 40 Gew.-%). Vorzugsweise wird diese Fraktion in die Hydroformylierungsstufe zurückgeführt.

Die den Ameisensäureester enthaltende Nachlauffraktion wird zusammen mit dem Olefin der Hydroformylierung unterworfen. Ihr Anteil im Einsatzmaterial kann bis zu 25 Gew.-%, bezogen auf das dem Reaktor zugeführte Olefin, betragen. Je größer der Anteil Ameisensäureester bzw. Ameisensäureester enthaltendes Gemisch im Olefin ist, desto geringer ist der Ameisensäureesterabbau in der Synthesestufe. Beispielsweise beträgt der Abbau unter sonst gleichen Bedingungen insbesondere bei gleich großem Wassergehalt bei Einsatz von 70 Gew.-% Olefin und 30 Gew.-% ameisensäureesterhaltigen Gemisch 50 %, bei Verdopplung des ameisensäureesterhaltigen Gemisches auf 60 % nur noch 30 %. Da ein zu hoher Ameisensäureestergehalt im Reaktionsprodukt die Qualität der aus der Nachlauffraktion isolierten Alkohole beeinträchtigt, ist die dem Olefin zuzusetzende Ameisensäureester-Menge durch die Qualitätsanforderungen an den Alkohol begrenzt.

Ein wesentliches Merkmal der erfindungsgemäßen Arbeitsweise ist die Gegenwart von Wasser in dem der Hydroformylierung unterworfenen Gemisch der Einsatzstoffe. Die Wassermenge richtet sich nach der Löslichkeit von Wasser in dem Reaktionsgemisch und wird hauptsächlich durch gesättigte Kohlenwasserstoffe negativ beeinflußt. Üblicherweise beträgt sie 2 bis 5 Gew.-%, bezogen auf das gesamte Einsatzmaterial.

Die Umsetzung der Einsatzstoffe wird unter den üblichen Bedingungen der Hydroformylierung durchgeführt. Man arbeitet bei 100 bis 250 °C, insbesondere 170 bis 180 °C, und 100 bis 300 bar, insbesondere 200 bis 280 bar. Kohlenmonoxid und Wasserstoff werden üblicherweise im Verhältnis 1 : 1 dem Reaktor zugeführt, jedoch ist es auch möglich, einen der beiden Komponenten im Überschuß zu verwenden, so daß das Verhältnis von Kohlenmonoxid und Wasserstoff 1 : 3 bis 3 : 1 betragen kann. Als Katalysator für die Reaktion kommt Kobalt in Betracht, das auch in Form von Verbindungen wie Kobaltsalze organischer Säuren eingesetzt werden kann. Im Verlauf der Reaktion bilden sich aus dem Metall oder aus den Salzen Kobaltcarbonylverbindungen, die die eigentlichen Katalysatoren der Reaktion sind. Zur Aufarbeitung des Reaktionsproduktes, dem die ameisensäureesterhaltige Fraktion zugesetzt war, wird zunächst der Katalysator abgetrennt und darauf das Reaktionsprodukt in seiner Gesamtheit hydriert. Dabei werden die im Gemisch enthaltenen Aldehyde zu den entsprechenden Alkoholen umgewandelt, die in einem weiteren Schritt destillativ gewonnen werden. Die Erfindung wird durch die folgenden Beispiele näher erläutert:

Beispiel 1

In ein Hochdruckreaktionsrohr, das sich auf einer Temperatur von 180 °C und unter einem Wassergasdruck (CO : H$_2$ = 1 : 1) von 300 bar befindet, werden stündlich 1.410 Gew.-Teile Diisobutylen, 35 Gew.-Teile Wasser und ein in Kohlenwasserstoffen lösliches Kobaltsalz eingepumpt. Das dem Reaktor laufend entnommene Reaktionsprodukt enthält neben Isononanal, Isononanol sowie einer geringen Menge weiterer Nebenprodukte 8,5 Gew.-% Isononylformiat.

Beispiel 2

In den gleichen Hochdruckreaktor wie in Beispiel 1, der sich auf einer Temperatur von 180 °C und unter einem Wassergasruck (CO : H$_2$ = 1 : 1) von 300 bar befindet, wird stündlich ein Gemisch aus 1.410 Gew.-Teilen Diisobutylen, 422 Gew.-Teilen einer Isononanalnachlauffraktion (43 Gew.-% Isononylformiat und 57 Gew.-% Isononanol) 60 Gew.-Teilen Wasser und ein in Kohlenwasserstoffen lösliches Kobaltsalz eingepumpt. Das dem Reaktor laufend entnommene Reaktionsprodukt enthält neben Isononanal, Isononanol sowie geringen Mengen weiterer Nebenprodukte nur 11 % Isononylformiat, obwohl nach der nach Beispiel 1 zu erwartenden Neubildung an Formiat und dem mit dem Einsatzprodukt eingeführten Isononylformiat ein Gemisch von etwa 18 Gew.-% an Isononylformiat zu erwarten gewesen wäre.

Vergleichsbeispiel

In den gleichen Hochdruckreaktor wie in Beispiel 1, der sich auf einer Temperatur von 180 °C und unter einem Wassergasdruck (CO : H$_2$ = 1 : 1) von 300 bar befindet, wird stündlich ein Gemisch aus 1.410 Gew.-Teilen Diisobutylen, 422 Gew.-Teilen einer Isononanalnachlauffraktion (43 Gew.-% Isononylformiat und 57 Gew.-% Isononanol) und ein in Kohlenwasserstoffen lösliches Kobaltsalz eingepumpt. Das dem Reaktor laufend entnommene Reaktionsprodukt enthält neben Isononanal, Isononanol sowie geringen Mengen weiterer Nebenprodukte 16,6 Gew.-% Isononylformiat. Ein solcher erhöhter Formiatgehalt ist in diesem Fall zu erwarten, da unter den wasserfreien Bedingungen kein Formiatabbau eintritt.

**Anspruch**

Verfahren zum Abbau von Ameisensäureestern oder Ameisensäureester enthaltenden Gemischen, die bei der Umsetzung von Olefinen mit 4 bis 12 Kohlenstoffatomen mit Kohlenmonoxid und Wasserstoff bei 100 bis 250 °C und 100 bis 300 bar in Gegenwart von Kobaltkatalysatoren erhalten werden und nach Abtrennung der Katalysatoren und der leichter flüchtigen Reaktionsprodukte, insbesondere der Aldehyde, als Nachlauffraktion anfallen, dadurch gekennzeichnet, daß

— die Ameisensäureester bzw. die Ameisensäureester enthaltenden Gemische in die Synthesestufe zurückgeführt und

— zusammen mit Olefin, Kohlenmonoxid und Wasserstoff in Gegenwart von 2 bis 5 Gew.-% Wasser, bezogen auf das Einsatzgemisch,

— bei Temperaturen von 100 bis 180 °C umgesetzt werden.

## Claim

Process for the decomposition of mixtures containing formic acid esters or formic acid ester, which are obtained by the reaction of olefines with 4 to 12 carbon atoms with carbon monoxide and hydrogen at 100 to 250 °C and 100 to 300 bar in the presence of cobalt catalysts, and after separation of the catalysts and the more easily volatile reaction products, especially the aldehydes, occur as an after-running fraction, characterised in that

— the mixtures containing formic acid esters or formic acid ester are fed back into the synthesis stage and

— react together with olefine, carbon monoxide and hydrogen in the presence of 2 to 5 wt. % water, based on the initial mixture,

— at temperatures of 100 to 180 °C.

## Revendication

Procédé pour la décomposition d'esters formiques ou de mélanges contenant des esters formiques, qui sont obtenus par la réaction d'oléfines en $C_4$-$C_{12}$ avec le monoxyde de carbone et l'hydrogène à 100-250 °C et sous 100-300 bars, en présence de catalyseurs au cobalt, et se forment comme fraction de queue après séparation des catalyseurs et des produits de réaction volatils, en particulier des aldéhydes, caractérisé en ce que

— l'on renvoie dans l'étape de synthèse les esters formiques ou les mélanges contenant des esters formiques et

— on les fait réagir avec l'oléfine, le monoxyde de carbone et l'hydrogène en présence de 2 à 5 % en poids d'eau, par rapport au mélange,

— à des températures de 100 à 180 °C.